(19) Europäisches Patentamt
European Patent Office
Office européen des brevets

(11) **EP 2 569 282 B1**

(12) **EUROPÄISCHE PATENTSCHRIFT**

(45) Veröffentlichungstag und Bekanntmachung des Hinweises auf die Patenterteilung:
**09.09.2015 Patentblatt 2015/37**

(21) Anmeldenummer: **11717589.3**

(22) Anmeldetag: **28.04.2011**

(51) Int Cl.:
***C07D 209/20*** *(2006.01)* ***C01D 15/02*** *(2006.01)*

(86) Internationale Anmeldenummer:
**PCT/EP2011/056718**

(87) Internationale Veröffentlichungsnummer:
**WO 2011/141294 (17.11.2011 Gazette 2011/46)**

(54) **VERFAHREN ZUR ABTRENNUNG VON TRYPTOPHAN**

METHOD FOR SEPARATING OFF TRYPTOPHAN

PROCÉDÉ DE SÉPARATION DE TRYPTOPHANE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.05.2010 DE 102010028916**

(43) Veröffentlichungstag der Anmeldung:
**20.03.2013 Patentblatt 2013/12**

(73) Patentinhaber: **Evonik Degussa GmbH 45128 Essen (DE)**

(72) Erfinder:
• **KESSLER, Christian 63741 Aschaffenburg (DE)**
• **BLÜMKE, Wilfried 61137 Schöneck (DE)**
• **LOTTER, Hermann 63674 Altenstadt (DE)**
• **POHLISCH, Joachim 63571 Gelnhausen (DE)**

(56) Entgegenhaltungen:
EP-A1- 2 108 423      WO-A1-98/32514
WO-A1-2009/105351     US-A- 5 071 560
US-A- 5 300 653

• **D. J. WU, Y. XIE, Z. MA, N. H. L. WANG: "Design of simulated moving bed chromatography for amino acid separations", IND. ENG. CHEM. RES., Bd. 37, 16. September 1998 (1998-09-16), Seiten 4023-4035, XP002663626,**

## Beschreibung

Die Erfindung betrifft ein Verfahren zur Abtrennung von Tryptophan aus wässrigen Stoffgemischen, insbesondere bereits teilweise aufgearbeiteten Fermentationsbrühen, mit Hilfe der simulierten Gegenstrom- oder SMB (simulated moving bed)-Chromatographie und eine Vorrichtung zur Durchführung des Verfahrens.

Stand der Technik

Tryptophan wird im Allgemeinen fermentativ, d.h. mit Hilfe von Mikroorganismen, produziert. Dies gilt im Besonderen für seine biologisch verwertbare L-Form. Nach der Fermentation wird die Biomasse abgetötet, abgetrennt und der flüssige Überstand weiterverarbeitet. Am Ende der Aufreinigungskette steht im Allgemeinen eine Kristallisation, bei der Tryptophan als hochreiner Feststoff durch Abtrennung von dem flüssigen Überstand, der sogenannten Mutterlauge, gewonnen wird. Diese ist gesättigt an Tryptophan und enthält des Weiteren noch Salze, andere Aminosäuren sowie weitere nicht näher definierte organische Komponenten, die während der Fermentation entstanden sind.

Liegen nach der Fermentation die ebenfalls aromatischen Aminosäuren Phenylalanin und Tyrosin in nicht zu vernachlässigenden Konzentrationen vor, gestaltet sich deren Abtrennung von Tryptophan aus der Mutterlauge aufgrund der ähnlichen physikalisch chemischen Eigenschaften als schwierig, so dass die Mutterlauge häufig verworfen wird.

US Patent 5,300,653 beschreibt die Abtrennung aromatischer Aminosäuren aus wässrigen Lösungen mittels Kationenaustauschchromatographie, ohne eine bestimmte Form der Prozessführung anzugeben. Die beiden aromatischen Aminosäuren Tryptophan und Phenylalanin konnten mit der dort beschriebenen Methode nicht getrennt werden.

In einer Veröffentlichung von Ribeiro et al. in Bioprocess Engineering 12 (1995) 95 - 102 wird die Abtrennung von Tryptophan aus Gemischen mit der aliphatischen Aminosäure Serin und Indol durch selektive Adsorption von Tryptophan an verschiedene Aktivkohlen und Absorbentien aus organischen Polymeren bei pH 8,0 beschrieben. Unter anderem wurde das Polymer XAD-7 (Rohm & Haas, Philadelphia, U.S.A.) verwendet. Ausgangsmaterial war eine Lösung aus Serin und Indol, aus der mit Hilfe immobilisierter Mikroorganismen Tryptophan produziert wurde. Das Trenngemisch enthielt außer diesen drei Substanzen keine weiteren Aminosäuren beziehungsweise aminosäureähnliche Komponenten. Die Gemischzusammensetzung ist zudem, im Unterschied zu fermentativ hergestellten Ausgangsmedien, keinen Schwankungen unterworfen. Die Trennung erfolgte diskontinuierlich. Als nicht-organische wasserbasierte Desorptionsmittel werden eine NaOH-Lösung (0,05 M) sowie eine HCl-Lösung (0,1 M) beschrieben. Eine Desorption mit reinem Wasser wird dort nicht erwähnt. Von einem anderen Adsorber (XAD-4,Rohm & Haas, Philadelphia, U.S.A.) konnte Tryptophan nur durch Zugabe eines organischen Lösungsmittels, beispielsweise Methanol oder Isopropanol, desorbiert werden.

In einer Veröffentlichung von Wu et al. in Industrial and Engineering Chemistry Research 37 (1998) 4023-4035 wird die Trennung eines definierten Zweistoffgemischs der aromatischen Aminosäuren Tryptophan und Phenylalanin ohne Temperaturgradient in Wasser beschrieben. Als Adsorber wurde ein PVP resin (Poly-4-vinylpyridin, Reillex HP polymer, Vertellus Specialties Inc, Indianapolis, U.S.A.) verwendet. Als Desorbens wurde Wasser verwendet. Die Trennung erfolgte analog zum bekannten 4-Zonen Simulated-Moving-Bed-Verfahrens mit einem geschlossenen internen Flüssigkeitskreislauf. Die Trennaufgabe wird durch das definierte Zweistoffgemisch in signifikantem Maße vereinfacht, da bei diesen, im Gegensatz zu fermentativ hergestellten Ausgangslösungen, keine Nebenreaktionen mit nicht definierten weiteren Verbindungen auftreten können.

Die Adsorption von Aminosäuren an neutrale polymere Adsorbentien wurde des Weiteren von Doulia et al. im Journal of Chemical Technology and Biotechnology 76 (2001) 83 - 89 beschrieben. Hier wurde beobachtet, dass die aromatischen Aminosäuren Tryptophan und Phenylalanin am stärksten an die zwei Adsorbermaterialien XAD-2 und XAD-4 (Rohm & Haas, Philadelphia, U.S.A.) binden. Eine Erhöhung der Ionenstärke in der Lösung führte zu einer verstärkten Adsorption. Allerdings wurden keine Angaben zur Desorption gemacht.

Aus der EP 1 106 602 B1 ist die Abtrennung einer basischen Aminosäure (L-Lysin) mit Hilfe des Verfahrens der simulierten Gegenstrom-Chromatographie aus Lösungen bekannt, die L-Lysin und weitere Verunreinigungen enthalten.

Die in Serie verbundenen Chromatographiesäulen sind mit einem starken Kationenaustauscher gefüllt.

Kationenaustauscher sind aber für eine Abtrennung von Tryptophan nicht geeignet, wenn die Lösungen zusätzlich Phenylalanin und/oder Tyrosin enthalten, da diese in vergleichbarer Weise mit einem Ionentauscher wechselwirken.

In US 5,071,560 wird die selektive Adsorption von Phenylalanin an das neutrale Polymer XAD-7 sowie dessen Desorption mit Wasser, Alkohol, Ketonen oder Estern beschrieben. In dieser Methode wird das zu adsorbierende Phenylalanin von weiteren, nicht mit dem Adsorber wechselwirkenden Komponenten getrennt und durch Desorption wieder von dem Adsorber gelöst. Verwendet wird eine Fermentationsbrühe, die neben Phenylalanin maßgeblich Salze, Milchsäure sowie weitere nicht näher definierte Aminosäuren enthält.

Die Grundzüge des SMB-Verfahrens wurden erstmals in dem US Patent 2,985,589 (1961) beschrieben. In den letzten Jahren wurde eine Vielzahl verschiedener Prozessvarianten erprobt. Übersichten finden sich beispielsweise in "Fundamentals of Preparative and Nonlinear Chromatography" von Guiochon et al. (Academic Press 2006, New York, U.S.A.) oder in Seidel-Morgenstern et al. Chemical Engineering and Technology 31 (2008) 826 - 837.

Ein SMB-Ansatz basierend auf wechselnden Lösungsmittelzusammensetzungen (Lösungsmittelgradient) mit drei aktiven Zonen sowie einem ausgelagerten Bereich zum Ausgleich von Gradientenschwankungen wurde beispielsweise von L. C. Keßler et al. im Journal of Chromatography A 1176 (2007)69 - 78 anhand der Reinigung von Antikörpern und Proteinen beschrieben. Maßgeblich für das Verfahren ist das Verwenden unterschiedlicher Salzkonzentrationen zur Erzielung unterschiedlicher Wechselwirkungsstärken. Als Desorptionsmittel dienten Natriumchlorid-Lösungen unterschiedlicher Konzentrationen.

Aufgabe der Erfindung ist es, ein Verfahren zur Abtrennung von Tryptophan aus wässrigen Stoffgemischen, insbesondere der nach der Kristallisation von Tryptophan aus Fermentationsbrühen anfallenden Mutterlaugen bereitzustellen und damit die Ausbeute der Tryptophanfermentation zu erhöhen.

Gegenstand der Erfindung ist ein Verfahren zur Abtrennung von gelöstem Tryptophan aus einem wässrigen, insbesondere weitere aromatische Aminosäuren enthaltenden Stoffgemisch mit Hilfe einer Variante der simulierten Gegenstrom- oder SMB-Chromatographie, bei der man in einem Trennungsabschnitt mit einer aus mehr als einer hintereinandergeschalteten, mit einem als Adsorbens geeigneten organischen Polymer gefüllten Säulen bestehenden Säulenanordnung, die in mehrere, bevorzugt drei funktionelle Zonen unterteilt ist,

a) das gelöstes Tryptophan enthaltende Stoffgemisch und Wasser als Desorbens an getrennten Stellen der Säulenanordnung kontinuierlich zuführt und

b) an einer zwischen diesen Zuläufen liegenden Stelle den mit Tryptophan angereicherten Extraktstrom und an einer weiteren, stromaufwärts vom Zulauf des Tryprophan enthaltenden Stoffgemischs gelegenen Stelle einen weitere Verbindungen aus dem eingesetzten Stoffgemisch enthaltenden Raffinatstrom getrennt abzieht, gegebenenfalls weiterverarbeitet und bevorzugt

c) die mit nicht desorbierten Verbindungen aus dem Stoffgemisch beladenen Säulen von diesen reinigt

dadurch gekennzeichnet, dass man einen Temperaturgradienten einstellt zwischen der Zone, in der das Desorbens eingeführt wird, und der Zone, aus der das Raffinat abgeleitet wird.

Angereichert bedeutet, dass der Extraktstrom Tryptophan in einer höheren Reinheit als in dem eingespeisten Stoffgemisch enthält.

In einer Ausführungsform handelt es sich bei dem Stoffgemisch insbesondere um die nach der Fermentation eines Tryptophan produzierenden Mikroorganismus anfallenden Fermentationsbrühen, die neben weiteren Verunreinigungen auch Phenylalanin und/oder Tyrosin enthalten und aus denen bevorzugt die Biomasse vorher abgetrennt wurde.

Bevorzugt setzt man das erfindungsgemäße Verfahren im Anschluss an die Kristallisation ein, bei der Tryptophan als hochreiner Feststoff gewonnen wird und von diesem der flüssige Überstand, die sogenannten Mutterlauge, abgetrennt wird. Diese ist gesättigt an Tryptophan und enthält des Weiteren noch Salze, andere Aminosäuren wie zum Beispiel Phenylalanin und/oder Tyrosin sowie weitere, nicht näher definierte und als Verunreinigungen wirkende Verbindungen, die während der Fermentation entstanden sind. Diese Mutterlauge wird in einer bevorzugten Variante mit dem beanspruchten Prozess aufgetrennt in

a) einen Produktstrom (Extrakt), in dem die überwiegende Menge des in der Mutterlauge vorhandenen Tryptophans vorliegt, und der gleichzeitig stark an Störstoffen abgereinigt ist, sowie

b) einen Abfallstrom (Raffinat), in dem die weiteren nicht erwünschten Mutterlaugenbestandteile vorliegen.

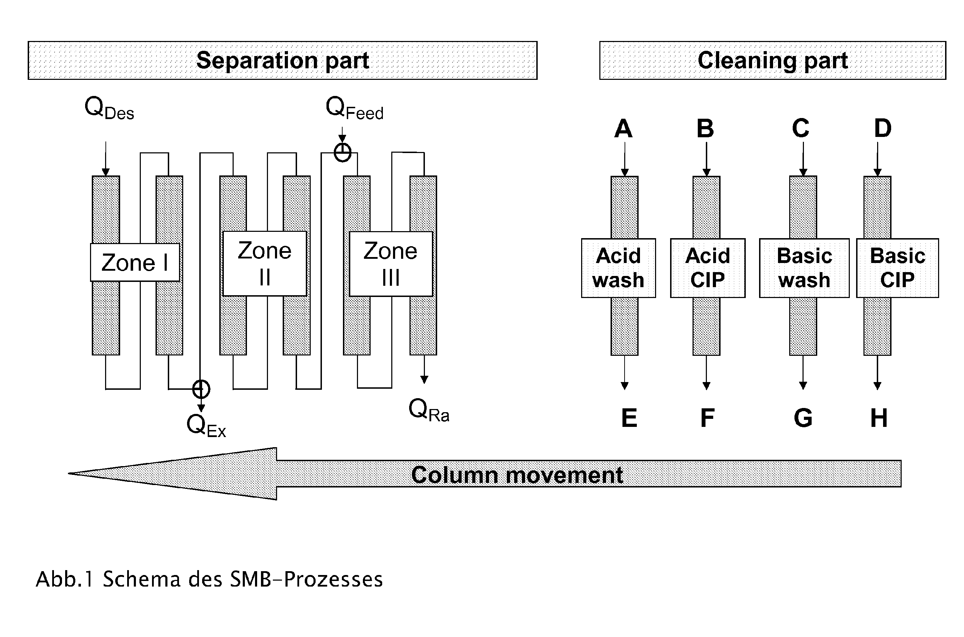

In Abbildung 1 ist das Schema des Prozesses wiedergegeben.

Der erhaltene Produktstrom wird anschließend wieder in den Hauptstrom des Aufarbeitungsprozesses der Fermentationsbrühe vor dem Kristallisationsschritt gegebenenfalls nach einer Aufkonzentration durch Einengung eingeschleust und so weiterverarbeitet. Ist der eingesetzte Strom eine zellfreie Fermentationslösung, kann der Produktstrom auch als solcher direkt weiterverarbeitet werden. Damit wird die Ausbeute der fermentativen Herstellung von Tryptophan erhöht. Dies ist Teil der Erfindung.

Das erfindungsgemäße Verfahren kann auch die beschriebene Kristallisation ersetzen.

Die Abtrennung geschieht mit Hilfe eines organischen Polymers, das durch eine mittlere Polarität charakterisiert ist. Wie in der vorliegenden Erfindung gezeigt werden konnte, ist dieses Material in der Lage, Tryptophan praktisch vollständig von den aromatischen Aminosäuren Phenylalanin und Tyrosin abzutrennen und gleichzeitig andere Verunreinigungen stark abzureichern. Zu diesen gehören im Besonderen auch UV-aktive Nebenprodukte ("UV-by products"), die bei einem Standardanalytikverfahren für Verunreinigungen nach European Pharmacopoeia 6.3 entweder vor ("UV-BP before") oder nach Tryptophan eluieren ("UV-BP after").

Als Adsorber sind besonders nichtionische polymere Adsorbentien geeignet, die Tryptophan reversibel so adsorbieren, dass ihre Wechselwirkung mit dem Tryptophan stärker ist als die mit den Verunreinigungen. Das stärker zurückgehaltene Tryptophan kann mit Wasser in einem bevorzugten Temperaturbereich von 20 bis ca. 80°C wieder desorbiert werden, wobei eine höhere Temperatur zu einer schnelleren Desorption und somit zu einer geringeren Zurückhaltung von Tryptophan führt. Ein Großteil der ebenfalls in den eingesetzten Fermentationsbrühen enthaltenen organischen Verunreinigungen wie z. B. Bruchstücke von Proteinen mikrobiellen Ursprungs oder Nebenprodukte wie Phenylalanin oder Tyrosin wechselwirken während des erfindungsgemäß ablaufenden Trennungsschritts nicht oder deutlich schwächer als Tryptophan mit dem Adsorbens, so dass diese im Durchlauf beziehungsweise im Raffinat zu finden sind. Wenige Verunreinigungen werden ebenfalls adsorbiert und bleiben auch bei der Desorption mit Wasser auf dem Adsorber haften.Diese werden in einem anschließenden Reinigungsschritt mit alkalischen bzw. sauren Lösungen desorbiert. Anschließend werden die so gereinigten Säulen wieder in den Trennprozess eingeschaltet. Geeignete Adsorbentien umfassen Polymere aus der Gruppe der Acryl/Methacrylgruppe enthaltenden Materialien und Polymere auf Polystyrolbasis.

Bevorzugte Adsorbentien sind Acrylpolymere wie z. B. XAD7, XAD7HP® (Rohm & Haas) oder HP2MG® (Diaion).

Die besonders geeigneten Adsorbentien zeigen

a) ein Rückgrat aus Acrylaten, bevorzugt Methacrylate, Acrylsäure und deren Derivate

b) eine aliphatische Quervernetzung, bevorzugt durch polyfunktionale Monomere

c) ein sich aus den beiden oben genannten Eigenschaften ergebendes Dipolmoment, das höher ist als bei ST-DVBbasierten Materialien

d) eine makroretikulare Porenstruktur

e) eine geeignet hohe Oberfläche

f) das Fehlen von Interaktion durch Ionenaustausch

Eine Belastung des Trennsystems mit organischen Lösungsmitteln erfolgt nicht, da man erfindungsgemäß für die Trennung nur Wasser, insbesondere entmineralisiertes Wasser, benötigt, das mit einer Temperatur von 20 bis ~ 98°C, bevorzugt 60 bis 70°C eingespeist wird. Dies ermöglicht eine einfache Prozessführung und vermeidet ein Eintragen von zusätzlichen Fremdstoffen. Die apparative Umsetzung erfolgt mit einer Variante des "Simulated Moving Bed"-Prozesses, die in Abbildung 1 dargestellt wird.

Die Säulen werden mit Hilfe von Ventilkonstruktionen, die auf dem Markt verfügbar sind, miteinander verschaltet und in einer Realisierungsform nach Ablauf einer bestimmten Zeit (der "Schaltzeit") mit Hilfe dieser Schaltung simultan im Kreis bewegt. Im vorliegenden Fall geschieht dies durch ein zentrales Schaltventil der Firma Knauer (Knauer Wissenschaftliche Gerätebau GmbH, Berlin). Das Verfahren kann jedoch auch mit anderen Ventilkonstruktionen, beispielsweise mit geeignet verschalteten Zwei-WegeVentilen realisiert werden. Der Feststoffgegenstrom wird bei diesem Verfahren durch eine Positionsveränderung der Zu- und Abläufe simuliert. In diesem Fall ist auch ein nicht-simultanes Weiterschalten der Zu- und Abläufe möglich gemäß US Patent 6,136,198. Eine weitere Möglichkeit ist das sequentielle Durchführen der im Folgenden beschriebenen Schritte etwa nach dem Sequential SMB-Prinzip (S. Baudouin und X. Lancrenon, Industries Alimentaires et Agricoles, 120, 2003, Seiten 42-48)). Prinzipiell lässt sich das Grundprinzip des erfindungsgemäßen Verfahrene an alle bereits bekannten Varianten der kontinuierlichen und diskontinuierlichen Chromatographie anpassen und stattet diese dadurch mit neuen Funktionalitäten aus.

Der Flüssigkeitsstrom durchläuft beim SMB-Betrieb mehrere mit Adsorbens gefüllte Festbettsäulen. Die erfindungsgemäße Vorrichtung wird durch die kontinuierliche Zugabe bzw. Entnahme der Feed-, Desorbens, Extrakt- und Raffinatströme in drei funktionelle Zonen unterteilt wie Abbildung 1 zeigt. Jede einzelne dieser Zonen übernimmt dabei eine spezielle Trenn- bzw. Aufarbeitungsfunktion. Vorteilhaft im Sinne der Erfindung ist bei Verwenden nicht genau definierter zu trennender Gemische ein offener Flüssigkeitskreislauf ("open loop"). Jede funktionelle Zone enthält jeweils ein bis mehrere chromatographische Säulen.

Desweiteren kann, bevorzugt unter Hinzufügung einer vierten Zone, die stromaufwärts von der Raffinatentnahmestelle liegt, auch ein geschlossener Flüssigkeitskreislauf realisiert werden.

Im erfindungsgemäß bevorzugt offenen Betrieb des SMB-Verfahrens existieren generell 3 interne ($Q_I$, $Q_{II}$ und $Q_{III}$) und 4 externe Mengenströme ($Q_{Feed}$, $Q_{Des}$, $Q_{Ex}$ und $Q_{Ra}$), die über folgende Bilanzgleichungen

$$Q_{I} = Q_{Des}$$

$$Q_{II} = Q_{I} - Q_{Ex}$$

$$Q_{III} = Q_{II} + Q_{Feed} = Q_{Ra}$$

miteinander verknüpft sind. Für die Festlegung des Betriebspunktes müssen die Massen- beziehungsweise Volumenströme sowie die Taktzeit $t_s$ derart spezifiziert werden, dass die Trennaufgabe gelöst und dadurch ein wirtschaftlich optimaler Betrieb bei Einhaltung der vorgegebenen Produkteinheiten erzielt wird. Die Taktzeit $t_s$ bestimmt dabei die "Geschwindigkeit" des scheinbaren Feststoffgegenstroms und damit maßgeblich den Trennerfolg. Die Festlegung der Ströme kann entsprechend dem Stand der Technik auf verschiedene Arten realisiert werden, wie etwa in "Fundamentals of Preparative and Nonlinear Chromatography" von Guiochon et al. (Academic Press 2006, New York, U.S.A.) beschrieben.

Der im SMB-Prozess erzeugte Gegenstrom wird genutzt, um Tryptophan von einer Mischfraktion der Verschmutzungen zu trennen. Grundlage sind die jeweils unterschiedlichen Wanderungsgeschwindigkeiten der Verbindungen, die dazu verwendet werden, Tryptophan sowie die Mischfraktion der Verschmutzungen ("neutral waste") zu zwei unterschiedlichen Auslässen zu dirigieren (Abbildung 1). Aus dem mit "Extract" bezeichneten Auslass lässt sich während des gesamten Prozesses der tryptophanhaltige Produktstrom abziehen, mit dem mit "Raffinat" bezeichneten Strom werden die Störstoffe entfernt. Weiterhin erfolgt eine kontinuierliche Zugabe von temperiertem Wasser über den "Desorbent"-Eingang. Der Trennerfolg wird durch eine geeignete Auswahl der Fließgeschwindigkeiten der Zu- und Abläufe sowie der Schaltzeit bestimmt.

Erfindungsgemäß wird in dem aus drei Zonen bestehenden Trennabschnitt ein Temperaturgradient eingehalten. Die Zufuhr des temperierten Wassers erfolgt mit einer im allgemeinen mit 10 bis 40°C höheren Temperatur als der der über den Feedeingang eingetragenen aufzutrennenden Lösung (Trenngemisch). Als vorteilhaft haben sich Zulauftemperaturen des Desorbens von mindestens 60 °C und von 45 °C im Trenngemisch herausgestellt.

Das als Desorbens eingesetzte Wasser ist bevorzugt vollentsalzt. Es ist aber auch möglich, Wasser mit einem Salzgehalt einzusetzen, bevorzugt von 0,01 bis 10 Gew.-%, insbesondere 0,01 bis 3 Gew.-%. Tryptophan wird auch unter diesen Bedingungen abgetrennt. Dabei ist allerdings in Kauf zu nehmen, dass der Extraktstrom mit diesem Salz belastet ist.

Es werden wässrigen Stoffgemische, insbesondere Lösungen, eingesetzt, die 0,1 bis 39 g/l, insbesondere 0,5 bis 38 g/l, besonders bevorzugt 10 bis 18 g/l Tryptophan enthalten.

Der Gehalt ist insbesondere abhängig davon, ob Mutterlaugen oder sonstige nicht abgereinigte Tryptophanlösungen aufgearbeitet werden sollen und bei welchem pH-Wert diese vorliegen.

Der pH-Wert der eingesetzten Gemische bzw. Lösungen reicht von 2 bis 9, insbesondere von 2,5 bis 7 und liegt besonders bei 5,8.

Bei dem erfindungsgemäßen Verfahren werden parallel zum Abtrennungsprozess die im regelmäßigen Durchlauf nicht für die Trennung benötigten Säulen in einem Reinigungsabschnitt ("cleaning part") mit verschiedenen Agenzien behandelt, um von dem Adsorbens stark daran bindende sowie schlecht lösliche Verunreinigungen zu entfernen.

Die Säulen werden hierfür zunächst mit einem geeigneten wässrigen basischen Reinigungsmittel behandelt. Geeignet, ohne hierauf beschränkt zu sein, sind im Besonderen Natriumhydroxid-Lösungen in Konzentrationen von 0,05 bis 1 M. Darüber hinausgehend können weitere Lösungen verwendet werden, die dem Stand der Technik nach in der Lage sind, das Adsorbens von stark bindenden Verunreinigungen zu befreien. Beispielhaft, ohne darauf beschränkt zu sein, können hier mit Wasser mischbare organische Lösungsmittel genannt werden, wie z. B. Ethanol, Methanol, Aceton oder Isopropanol.

In einem weiteren parallelen Prozessschritt werden die bereits mit dem ersten Reinigungsmittel behandelten Säulen mit einem geeigneten Medium gespült. Dieses ist im Besonderen entsalztes Wasser, doch auch andere wässrige Lösungen mit Pufferwirkung können verwendet werden. Bei einem geeigneten Reinigungsmittel kann auch auf den Spülschritt vor der zweiten Reinigung verzichtet werden.

In einem weiteren parallelen Schritt werden die bereits mit dem ersten Reinigungsmittel behandelten und optional gespülten Säulen mit einem zweiten Reinigungsmedium in Kontakt gebracht. Besonders geeignet ist hier eine saure wässrige Lösung, beispielsweise 0,01 bis 0,1, insbesondere 0,05 M Schwefelsäure. Darüber hinaus können auch andere Reinigungsmedien verwendet werden, die dem Stand der Technik nach in der Lage sind, im neutralen beziehungsweise basischen Millieu schwerlösliche Substanzen besser zu lösen.

In einem weiteren parallelen Schritt werden die bereits mit den beiden Reinigungsmitteln behandelten Säulen entweder erneut oder zum ersten Mal mit einem geeigneten wässrigen Medium gespült. Dieses ist im Besonderen entsalztes Wasser, doch auch andere wässrige Lösungen können verwendet werden.

In einer bevorzugten Form der Erfindung werden alle Reinigungsschritte kontinuierlich und parallel zur laufenden Trennung durchgeführt.

In einer weiteren Realisierungsform können einzelne Teilschritte weggelassen werden.

In einer weiteren Realisierungsform können alle oder nur ausgewählte Reinigungsschritte auch entkoppelt von der Trennung durchgeführt werden. So ist beispielsweise eine Durchführung der Reinigung in einem nur täglichen oder an eine bestimmte Trennungslaufzeit gekoppelten Rhythmus möglich.

Offenbart wird auch eine Vorrichtung zur Abtrennung von gewünschten organischen Verbindungen aus einem wässrigen Stoffgemisch mit Hilfe der simulierten Gegenstrom- oder SMB-Chromatographie, mit einer aus mehr als einer hintereinandergeschalteten, mit einem

Adsorbens gefüllten Säulen bestehenden Säulenschaltung, die in drei funktionelle Zonen unterteilt ist, in denen die folgenden Aufgaben realisiert werden

Zugabe des Feedstroms,

Entnahme des Eluatstromes,

Entnahme des Raffinatstroms,

Zugabe des Desorbens,

und in denen zwischen den Zonen I und III ein Temperaturgradient von 10 bis 40 °C, insbesondere 15 bis _25 °C gemäß Abbildung 1, hervorgerufen durch die unterschiedlichen Zulauftemperaturen im Desorbens und Feed eingestellt ist. Der Temperaturgradient begünstigt die Trennung und verringert den Verbrauch an Desorbens. Er ist für ein Gelingen der Trennung jedoch nicht zwingend vorausgesetzt.

Dieser Aufbau unterscheidet sich von den bekannten Vorrichtungen dadurch, dass im Trennabschnitt Chromatographiesäulen in einer 3-Zone-Anordnung mit einem offenen Flüssigkeitskreislauf und einem Temperaturgradienten angeordnet sind und dieser Anlagenteil mit einem Reinigungsabschnitt ("cleaning part") gekoppelt ist, der Säulen derselben Adsorbensfüllung enthält, die im Wechsel mit denen aus der Trennabschnitt von den anhaftenden Verunreinigungen gereinigt werden.

Die Zahl der Säulen in den Zonen reicht im Allgemeinen von mehr als einer bis 32, insbesondere 16. Jede Zone enthält mindestens eine Säule. Deren Gesamtzahl und Verteilung über die Zonen kann, angepasst an die Temperatur, die Tryptophankonzentration, die gewünschte Wanderungsgeschwindigkeit und die Reinheit des Produkts, durch Standardversuche ermittelt werden. Die Säulenzahl wird weiterhin auch durch die Art der Prozessführung und die Umsetzung des SMB-Verfahrens beeinflusst.

Mit dem erfindungsgemäßen Verfahren konnten in Laborversuchen, ausgehend von Mutterlaugen mit Tryptophankonzentrationen zwischen 9 und 22 g/kg Mutterlauge und Reinheiten zwischen 7 und 15% (bezogen auf die Gesamttrockenmasse), Produktströme mit einer mittleren Reinheit von mehr als 85%, einer mittleren Tryptophanausbeute von besser als 85% sowie einer Tryptophankonzentration von 4 bis 5 g/kg im Extrakt erzielt werden.

Damit gelingt es, die in der Mutterlauge enthaltenen Trypthophanmengen zu einem hohen Prozentsatz in gereinigter Form abzutrennen und in wirtschaftlicher Weise in den Aufarbeitungsprozess der Fermentationsbrühe zur Verbesserung der Gesamtausbeute zurückzuführen.

Beschreibung Abb. 1

Abb. 1 gibt einen schematischen Aufbau und die Säulenanordnung des erfindungsgemäß eingesetzten SMB-Prozesses wieder.

Die Anordnung ist in einen Trennungs- (separation part) und einen Reinigungsteil (cleaning part) aufgeteilt.

Im Trennungsteil wird beispielsweise der Desorbentstrom $Q_{Des}$ mit 60° C und das aufzutrennende, wässrige Gemisch $Q_{Feed}$ mit 45° C eingespeist und das Raffinat $Q_{Ra}$ und der Extrakt $Q_{Ex}$, in dem das Tryptophan abgetrennt vorliegt, abgezogen.

Im Reinigungsteil erfolgt beispielsweise an den Stellen A und B die Zuführung von Wasser, an der Stelle B die Zuführung von 0,05 M Schwefelsäure sowie an der Stelle D die Zuführung von 0,5 M NaOH.

Nach dem Durchlauf durch die zu reinigenden Säulen treten an der Stelle E gebrauchte Schwefelsäure, an der Stelle F eine Lösung leichter gebundener, wasserlöslicher Verunreinigungen, an der Stelle G gebrauchte NaOH und der Stelle H eine Lösung mit den stark gebundenen Verunreinigungen aus. (CIP bedeutet: cleaning in place).

**Beispiele**

1. Verfahrensparameter

Verwendeter Adsorber: Amberlite XAD-7 HP (Rohm & Haas) Säulendimensionen (d x L): 1,5 * 15 cm

SMB-Anlage: Knauer CSEP 916 (Knauer Wissenschaftliche Gerätebau GmbH, Berlin, Deutschland)
Pumpen: Watson-Marlow U101 (Wilmington, MA, U.S.A.) Zu trennendes Gemisch: Kristallisationsmutterlauge:

| Stoff | Konzentrationsbereich [g/kg] |
|---|---|
| Tryptophan | 12 - 22 |
| Tyrosin | 1,2 - 2,1 |
| Phenylalanin | 1,1 - 4,8 |
| Weitere Nebenprodukte | 220 - 260 |

Von den genannten Parametern sind die weiteren unabhängig, wie z. B. die betrachteten Schaltzeiten, die zwischen 8 und 14 Minuten lagen. Es gibt jedoch keinen Grund, diese einzuschränken. Die in der Laboranlage verwendeten Ströme lagen zwischen 0,07 und 0,33 kg/h, ohne darauf beschränkt zu sein, limitiert durch die verwendeten Schlauchpumpen. Auch hier kann jeder Pumpentyp verwendet werden, der in der Lage ist, die für den zu erzielenden Durchsatz benötigten und eine Trennung sicherstellenden Fließgeschwindigkeiten, zu liefern.

Die verwendeten Temperaturen lagen zwischen Raumtemperatur (25°C) und 60°C, als vorteilhaft herausgestellt haben sich eine Temperatur des Desorbens von mindestens 60 °C, jedoch nicht höher als 80°C, sowie 45°C für die verbleibenden Ströme und Säulen.

2. Anwendungsbeispiele:

2.1 Bestimmung des Adsorptionsverhaltens

Im Rahmen einer Messkampagne wurde das Ad- und Dersoptionsverhalten von Tryptophan, Phenylalanin und Tyrosin mit einer der in Abschnitt 1 beschriebenen Säulen untersucht. Hierfür wurden 100 $\mu$L der Probe in einen die Säule durchströmenden Wasserstrom injiziert und die Austrittszeit der Aminosäuren am Ausgang detektiert. Es konnte gezeigt werden, dass Tryptophan unter den aromatischen Aminosäuren sowohl in neutralem, leicht saurem, als auch in saurem Milieu am stärksten zurückgehalten wird. Eine Erhöhung der Temperatur führte beim Tryptophan zu einer Verkürzung der Retentionszeit:

Retentionszeit für Tryptophan bei pH 5,9 (in Minuten)

| | 45 °C | 60 °C |
|---|---|---|
| **5 g/kg** | 37,3 | 31,9 |
| **10 g/kg** | 37,1 | 31,1 |
| **17 g/kg** | - | 33,5 |

Retentionszeiten für Phenylalanin bei pH 5,9

| | 45 °C | 60 °C |
|---|---|---|
| **5 g/kg** | 15,6 | 15,3 |
| **10 g/kg** | 15,2 | 15,1 |

Die Retentionszeiten für Tyrosin lagen auf demselben Niveau wie für Phenylalanin. Bei einem pH-Wert von 2,5, 45 °C und einer Konzentration von 10 g/kg lag die Retentionszeit für Tryptophan bei 39,1 Minuten, für Phenylalanin bei gleichen Bedingungen bei 15,8 Minuten. Eine Trennung ist somit auch bei saurem pH-Wert möglich.

2.2 Erste SMB-Experimentalkampagne

Hier wurden drei Arbeitspunkte experimentell überprüft. Als Einsatzstoffe dienten verschiedene Chargen von tryptophanhaltiger Prozessmutterlauge sowie die in Abschnitt 1 beschriebenen Lösungen. Die Bedingungen waren:

|  | $Q_{DeS}$ | $Q_{Feed}$ | $Q_{Ex}$ | $Q_{Ra}$ | $t_S$ |
|---|---|---|---|---|---|
| *Arbeitspunkt 1* | 5.49 g/min | 1.18 g/min | 3.21 g/min | 3.47 g/min | 14.0 min |
| *Arbeitspunkt 2* | 5.74 g/min | 1.26 g/min | 3.30 g/min | 3.69 g/min | 12.9 min |
| *Arbeitspunkt 3* | 5.59 g/min | 1.27 g/min | 3.26 g/min | 3.60 g/min | 12.75 min |

Die Temperatur des Desorbens betrug 60°C, die der Säulen sowie der anderen Lösungen 45°C. Die Ergebnisse der Versuche waren:

| | TRP Reinheit (Trocken-masse) [%] | TRP Ausbeute [%] | Entfernung von PHE [%] | Entfernung von TYR [%] | Entfernung von UV BP before [%] | Entfernung von UV BP after [%] |
|---|---|---|---|---|---|---|
| *Arbeitspunkt 1* | 87.1 | 37 | 100 | 100 | n.m. | n.m. |
| *Arbeitspunkt 2* | 82.2 | 76 | 100 | 100 | 92.4 | 34.9 |
| ***Arbeits-punkt 3*** | **87.6** | **85** | **99.1** | **96.1** | **93.5** | **41.8** |

2.3 12-Tage Versuch

In einer weiteren Kampagne wurde die Langzeitstabilität evaluiert. Über einen Versuchszeitraum von 12 Tagen wurde der Prozess mit den folgenden Parametern betrieben:

| $Q_{Des}$ | $Q_{Feed}$ | $Q_{Ex}$ | $Q_{Ra}$ | $t_S$ |
|---|---|---|---|---|
| 5.6 g/min | 1.3 g/min | 3.26 g/min | 3.60 g/min | 12.75 min |
| $Q_{NaOH}$ | $Q_{BasicWash}$ | $O_{H2SO4}$ | $Q_{AcidWash}$ | |
| 2.35 g/min | 1.91g/min | 2.89 g/min | 2.02 g/min | |

Als Einsatzstoffe dienten Mutterlaugen aus der Tryptophankristallisation. Im Laufe des Prozesses konnten durchschnittliche Reinheiten von 89,3 % bezogen auf die Gesamttrockenmasse) sowie Ausbeuten von 90,4 % (bezogen auf die eingetragene Tryptophanmasse) erzielt werden. Die Konzentrationen von Phenylalanin und Tyrosin konnten im Mittel auf 0,002 bzw. 0,001 g/l reduziert werden. Eine Rückführung in die Tryptophankristallisation konnte erfolgreich realisiert werden und zeigte keine negativen Effekte.


**Patentansprüche**

1.   Verfahren zur Abtrennung von Tryptophan aus einem wässrigen Stoffgemisch mit Hilfe der simulierten Gegenstrom- oder SMB-Chromatographie, bei dem man in einem Trennungsabschnitt mit einer aus mehr als einer hintereinandergeschalteten, mit einem organischen als Adsorbens geeigneten Polymer gefüllten Säulen bestehenden Säulenanordnung, die in mehrere funktionelle Zonen unterteilt ist,

   a) das Tryptophan enthaltende Stoffgemisch und Wasser als Desorbens an getrennten Stellen der Säulenanordnung kontinuierlich zuführt und
   b) an einer zwischen diesen Zuläufen liegenden Stelle den mit Tryptophan angereicherten Extraktstrom und an einer weiteren, stromaufwärts vom Zulauf des Tryptophan enthaltenden Stoffgemischs gelegenen Stelle einen weitere Verbindungen aus dem eingesetzten Stoffgemisch enthaltenden Raffinatstrom getrennt abzieht, den Extraktstrom gegebenenfalls weiterverarbeitet und bevorzugt
   c) die mit nicht desorbierten Verbindungen aus dem Stoffgemisch beladenen Säulen von diesen reinigt
   **dadurch gekennzeichnet, dass** man einen Temperaturgradienten einstellt zwischen der Zone, in der das Desorbens eingeführt wird, und der Zone, aus der das Raffinat abgeleitet wird.

2.   Verfahren nach Anspruch 1, bei dem man das bevorzugt entsalzte Wasser mit einer Temperatur von 20 bis 98°C zuführt.

3.   Verfahren nach Anspruch 1, bei dem man ein Stoffgemisch einsetzt, dass Tryptophan in einer Konzentration von 0,1 bis 39 g/l enthält.

4.   Verfahren nach Anspruch 1, bei dem man ein Stoffgemisch einsetzt, das einen pH-Wert von 2,5 bis 9 aufweist.

5.   Verfahren gemäß den Ansprüchen 1 bis 4, bei dem man ein durch Fermentation erhaltenes Stoffgemisch einsetzt, das neben Trypthophan auch Phenylalanin und/oder Tyrosin enthält.

6.   Verfahren gemäß Anspruch 5, bei dem man als Stoffgemisch die Mutterlauge einsetzt, die bei der Kristallisation von Tryptophan aus der Fermentationsbrühe anfällt.

7.   Verfahren gemäß Anspruch 1, bei dem man als Adsorbens ein nicht ionisches Polymer einsetzt, das Tryptophan reversibel adsorbiert und von dem das Tryptophan mit Wasser im Temperaturbereich von 10 bis ca. 98°C desorbiert wird.

8.   Verfahren gemäß den Ansprüchen 1 bis 6, bei dem man Adsorbentien, ausgewählt aus der Gruppe XAD7®, XAD7HP® und HP2MG® einsetzt.

**9.** Verfahren gemäß den Ansprüchen 1 bis 8, bei dem man die Trennung kontinuierlich ablaufen lässt.

**10.** Verfahren gemäß den Ansprüchen 1 bis 8, bei dem man die Trennung semi-kontinuierlich ablaufen lässt.

**11.** Verfahren gemäß den Ansprüchen 1 bis 11, bei dem parallel zur Abtrennung des Tryptophan die in Abhängigkeit von der Taktzeit aus der Auftrennung abgeschalteten Säulen der Anordnung im Anschluss daran in einem Reinigungsabschnitt mit desorbierenden Verbindungen behandelt werden.

**12.** Verfahren gemäß den Ansprüchen 1 bis 12, bei dem in dem Reinigungsabschnitt das Adsorbens in einem folgende Schritte umfassenden Verfahren behandelt wird, wobei man

a) die Säulen hierfür zunächst mit einem geeigneten basischen wässrigen Reinigungsmittel behandelt, insbesondere Natriumhydroxid-Lösungen in Konzentrationen von 0,05 bis 1 M, gegebenenfalls kombiniert
b) in einem weiteren parallelen Prozesschritt gegebenenfalls die gemäß a) behandelten Säulen mit einem wässrigen Medium spült, ausgewählt aus bevorzugt entsalztem Wasser, oder wässrigen Lösungen mit Pufferwirkung,
c) in einem weiteren parallelen Schritt die gemäß a) und b) behandelten Säulen mit einem zweiten wässrigen Reinigungsmedium in Kontakt bringt,. insbesondere mit einer sauren wässrigen Lösung, bevorzugt 0,01 bis 0,05 M Schwefelsäure, und
d) in einem weiteren parallelen Schritt die behandelten Säulen entweder gegebenenfalls mit einem wässrigen Medium spült, ausgewählt aus bevorzugt entsalztem Wasser, oder wässrigen Lösungen die als Puffer verwendet werden können.

**13.** Verfahren gemäß den Ansprüchen 1 bis 11, bei dem man den das Tryptophan enthaltenden Extraktstrom einem durch Fermentation erhaltenen Tryptophan enthaltenden wässrigen Stoffgemisch zusetzt, bevor das Tryptophan daraus auskristallisiert wird.

**14.** Verfahren gemäß Anspruch 13, bei dem alle oder Teile der beschriebenen Reinigungsschritte gemäß Anspruch 13 entkoppelt von der Abtrennung in einem festgelegten Rhythmus durchgeführt werden.

**Claims**

**1.** Method for separating off tryptophan from an aqueous mixture of matter using simulated countercurrent chromatography or SMB chromatography in which, in a separation section having a column arrangement consisting of more than one series-connected columns packed with an organic polymer suitable as adsorbent, which column arrangement is subdivided into a plurality of functional zones,

a) the tryptophan-containing mixture of matter and water as desorbent are continuously fed to the column arrangement at separate points and
b) at a point situated between these feeds, the tryptophan-enriched extract stream, and at a further point situated upstream of the feed of tryptophan-containing mixture of matter, a raffinate stream containing further compounds from the mixture of matter used, are taken off separately, the extract stream optionally processed further and preferably
c) the columns that are loaded with non-desorbed compounds from the mixture of matter are cleared of said compounds
**characterized in that** a temperature gradient is established between the zone in which the desorbent is introduced and the zone from which the raffinate is conducted.

**2.** Method according to Claim 1, in which the preferably desalted water is fed at a temperature of from 20 to 98°C.

**3.** Method according to Claim 1, in which a mixture of matter is used that contains tryptophan at a concentration from 0.1 to 39 g/l.

**4.** Method according to Claim 1, in which a mixture of matter is used that has a pH from 2.5 to 9.

**5.** Method according to Claims 1 to 4, in which a mixture of matter obtained by fermentation is used that, in addition to tryptophan, also contains phenylalanine and/or tyrosine.

6. Method according to Claim 5, in which the mixture of matter used is the mother liquor which occurs on crystallization of tryptophan from the fermentation broth.

7. Method according to Claim 1, in which the adsorbent used is a non-ionic polymer that reversibly adsorbs tryptophan and from which the tryptophan is desorbed with water in the temperature range from 10 to approximately 98°C.

8. Method according to Claims 1 to 6, in which adsorbents selected from the group XAD7®, XAD7HP® and HP2MG® are used.

9. Method according to Claims 1 to 8, in which the separation proceeds continuously.

10. Method according to Claims 1 to 8, in which the separation proceeds semi-continuously.

11. Method according to Claims 1 to 10, in which, in parallel to separating off the tryptophan, the columns of the arrangement that are switched out of the separation, in dependence on the cycle time, are treated subsequently thereto with desorbing compounds in a cleaning section.

12. Method according to Claims 1 to 11, in which, in the cleaning section, the adsorbent is treated in a method comprising the following steps, wherein

a) the columns for this purpose are first treated with a suitable basic aqueous cleaning agent, in particular sodium hydroxide solutions at concentrations from 0.05 to 1 M, optionally combined
b) in a further parallel process step optionally the columns treated according to a) are flushed with an aqueous medium selected from preferably desalted water, or aqueous solutions having a buffer action,
c) in a further parallel step the columns that are treated according to a) and b) are contacted with a second aqueous cleaning medium, in particular with an acidic aqueous solution, preferably 0.01 to 0.05 M sulfuric acid, and
d) in a further parallel step the treated columns are either optionally flushed with an aqueous medium, selected from preferably desalted water, or aqueous solutions that can be used as a buffer.

13. Method according to Claims 1 to 11, in which the extract stream containing the tryptophan is added to an aqueous mixture of matter containing tryptophan obtained by fermentation before the tryptophan is crystallized out therefrom.

14. Method according to Claim 13, in which all or some of the described cleaning steps as claimed in claim 13 are carried out in an established rhythm decoupled from the separation.

**Revendications**

1. Procédé pour la séparation de tryptophane à partir d'un mélange aqueux de substances à l'aide de la chromatographie SMB ou à contre-courant simulé, dans lequel, dans une section de séparation à l'aide d'un système de colonnes consistant en plusieurs colonnes raccordées en série, garnies d'un polymère organique approprié en tant qu'adsorbant, système qui est divisé en plusieurs zones fonctionnelles,

a) on introduit en continu en des points distincts du système de colonnes le mélange de substances contenant du tryptophane, et de l'eau en tant que désorbant et
b) on décharge séparément en un point situé entre ces alimentations le courant d'extrait enrichi en tryptophane et en un autre point placé en amont de l'arrivée du mélange de substances contenant du tryptophane un courant de raffinat contenant d'autres composés provenant du mélange de substances introduit, éventuellement on soumet le courant d'extrait à un traitement ultérieur et de préférence
c) on nettoie les colonnes chargées de composés non désorbés provenant du mélange de substances, pour l'élimination de ceux-ci
**caractérisé en ce qu'**on établit un gradient de température entre la zone dans laquelle est introduit le désorbant et la zone de laquelle est évacué le raffinat.

2. Procédé selon la revendication 1, dans lequel on introduit à une température de 20 à 98 °C l'eau de préférence déminéralisée.

3. Procédé selon la revendication 1, dans lequel on utilise un mélange de substances qui contient du tryptophane à une concentration de 0,1 à 39 g/l.

4. Procédé selon la revendication 1, dans lequel on utilise un mélange de substances qui présente un pH de 2,5 à 9.

5. Procédé selon les revendications 1 à 4, dans lequel on utilise un mélange de substances obtenu par fermentation, qui en plus de tryptophane contient également de la phénylalanine et/ou de la tyrosine.

6. Procédé selon la revendication 5, dans lequel on utilise comme mélange de substances la liqueur mère qui est produite lors de la cristallisation du tryptophane à partir du bouillon de fermentation.

7. Procédé selon la revendication 1, dans lequel on utilise comme adsorbant un polymère non ionique qui adsorbe réversiblement le tryptophane et est duquel le tryptophane est désorbé avec de l'eau dans la plage de température de 10 à environ 98 °C.

8. Procédé selon les revendications 1 à 6, dans lequel on utilise des adsorbants choisis dans le groupe constitué par XAD7®, XAD7HP® et HP2MG®.

9. Procédé selon les revendications 1 à 8, dans lequel on fait se dérouler la séparation en continu.

10. Procédé selon les revendications 1 à 8, dans lequel on fait se dérouler la séparation en mode semi-continu.

11. Procédé selon les revendications 1 à 10, dans lequel parallèlement à la séparation du tryptophane les colonnes du système mises hors du circuit de séparation en fonction du cycle de production sont à la suite de la séparation traitées par des composés désorbants dans une section de nettoyage.

12. Procédé selon les revendications 1 à 11, dans lequel dans la section de nettoyage on traite l'adsorbant dans un procédé comprenant les étapes suivantes, en

   a) traitant pour cela les colonnes d'abord par un produit aqueux basique de nettoyage approprié, en particulier des solutions d'hydroxyde de sodium à des concentrations de 0,05 à 1 M, éventuellement en combinaison
   b) dans une autre étape parallèle de processus, éventuellement en rinçant avec un milieu aqueux, choisi parmi l'eau de préférence déminéralisée ou des solutions aqueuses à effet tampon, les colonnes traitées selon a),
   c) dans une autre étape parallèle en mettant les colonnes traitées selon a) et b) en contact avec un deuxième milieu aqueux de nettoyage, en particulier avec une solution aqueuse acide, de préférence d'acide sulfurique 0,01 à 0,05 M, et
   d) dans une autre étape parallèle éventuellement en rinçant les colonnes traitées, avec un milieu aqueux, choisi parmi soit l'eau de préférence déminéralisée, soit des solutions aqueuses qui peuvent être utilisées comme tampon.

13. Procédé selon les revendications 1 à 11, dans lequel on ajoute au courant d'extrait contenant le tryptophane un mélange aqueux de substances contenant du tryptophane obtenu par fermentation, avant de faire cristalliser le tryptophane à partir de celui-ci.

14. Procédé selon la revendication 13, dans lequel on effectue la totalité ou des parties des étapes de nettoyage décrites selon la revendication 13 en les dissociant de la séparation en un rythme fixé.

Abb.1 Schema des SMB-Prozesses

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5300653 A
- EP 1106602 B1
- US 5071560 A
- US 2985589 A
- US 6136198 A

**In der Beschreibung aufgeführte Nicht-Patentliteratur**

- **RIBEIRO et al.** *Bioprocess Engineering,* 1995, vol. 12, 95-102
- **WU et al.** *Industrial and Engineering Chemistry Research,* 1998, vol. 37, 4023-4035
- **DOULIA et al.** *Journal of Chemical Technology and Biotechnology,* 2001, vol. 76, 83-89
- **GUIOCHON et al.** Fundamentals of Preparative and Nonlinear Chromatography. Academic Press, 2006
- **SEIDEL-MORGENSTERN et al.** *Chemical Engineering and Technology,* 2008, vol. 31, 826-837
- **L. C. KEßLER et al.** *Journal of Chromatography A,* 2007, vol. 1176, 69-78
- **S. BAUDOUIN ; X. LANCRENON.** *Industries Alimentaires et Agricoles,* 2003, vol. 120, 42-48